(19) Europäisches Patentamt
European Patent Office
Office européen des brevets

(11) Veröffentlichungsnummer : **0 367 939 B1**

(12) # EUROPÄISCHE PATENTSCHRIFT

(45) Veröffentlichungstag der Patentschrift : **01.04.92 Patentblatt 92/14**

(51) Int. Cl.$^5$ : **A61K 7/08,** A61K 7/06

(21) Anmeldenummer : **89116605.0**

(22) Anmeldetag : **08.09.89**

(54) **Lagerstabiles Haarkonditionierungsmittel mit Perlglanz.**

(30) Priorität : **08.11.88 DE 3837860**

(43) Veröffentlichungstag der Anmeldung : **16.05.90 Patentblatt 90/20**

(45) Bekanntmachung des Hinweises auf die Patenterteilung : **01.04.92 Patentblatt 92/14**

(84) Benannte Vertragsstaaten : **AT BE CH DE ES FR GB GR IT LI NL SE**

(56) Entgegenhaltungen :
**EP-A- 0 183 077**
**EP-A- 0 268 992**
**FR-A- 1 553 792**

(56) Entgegenhaltungen :
**US-A- 4 438 096**
**PATENT ABSTRACTS OF JAPAN, Band 11, Nr. 365 (C-460)[2812], 27. November 1987; & JP-A-62 138 412 (LION CORP.) 22-06-1987 (Kat. D)**

(73) Patentinhaber : **Wella Aktiengesellschaft**
**Berliner Allee 65**
**W-6100 Darmstadt (DE)**

(72) Erfinder : **Grundmann, Karin**
**Robert-Koch-Strasse 7**
**W-2900 Oldenburg (DE)**
Erfinder : **Lang, Günther, Dr.**
**Auf der Roten Erde 10B**
**W-6107 Reinheim 5 (DE)**
Erfinder : **Gross, Paul**
**Forstweg 54**
**W-6100 Darmstadt (DE)**

**EP 0 367 939 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist ein lagerstabiles, perlglänzendes Haarkonditionierungsmittel auf der Basis von Glycerinmonolaurinsäureester, Fettalkoholen und bestimmten quaternären Verbindungen.

Aus der eigenen DE-A- 3 440 935 sind bereits bestimmte Perlglanz besitzende Haarkonditionierungsmittel auf der Basis von Fettalkoholen und bestimmten quaternären Verbindungen bekannt. Diese Mittel enthalten jedoch Kokosfettsäuremonoethanolamid, das, wie neuere Untersuchungen gezeigt haben, unter ungünstigen Bedingungen cancerogen wirkende Nitrosamine bilden kann.

Ebenfalls sind aus der JP-A- 62-138 412 Haarkonditionierungsmittel bekannt, die (i) einen Glycerinfettsäureester (vorzugsweise ein Gemisch aus Glycerinmono- und Glycerindistearat sowie gegebenenfalls Glycerintristearat), (ii) einen Fettalkohol, (iii) bestimmte quaternäre Ammoniumsalze sowie (iv) einen polyoxyalkylierten Rizinusölfettsäureester enthalten. Die dort beschriebenen Mittel haben zwar zufriedenstellende haarkonditionierende und anwendungstechnische Eigenschaften; sie weisen jedoch keinen stabilen Perlglanz auf.

Es bestand daher die Aufgabe, ein perlglänzendes, kationisches Haarkonditionierungsmittel zur Verfügung zu stellen, das keine Fettsäureethanolamide enthält, physiologisch und dermatologisch unbedenklich ist und eine gute Lagerstabilität besitzt, das heißt bei dem unter den für solche Mittel üblichen Lagerbedingungen, insbesondere bei 20 bis 40 Grad Celsius, während einer mehrmonatigen, insbesondere 3 bis 6-monatigen, Lagerung keine Phasentrennung (Separation) auftritt.

Es wurde nunmehr gefunden, daß diese Aufgabe durch ein Haarkonditionierungsmittel, welches einen Glycerinmonolaurinsäureester, bestimmte quaternäre Verbindungen sowie $C_{10}$- bis $C_{18}$-Fettalkohole in einem definierten Mengenverhältnis enthält, in hervorragender Weise gelöst wird.

Gegenstand der vorliegenden Erfindung ist daher ein lagerstabiles, nicht-separierendes Haarkonditonierungsmittel mit Perlglanz auf der Basis eines Glycerinfettsäureesters, einer quaternären Verbindung sowie eines Fettalkohols, bestehend aus

(A) 0,2 bis 10 Gewichtsprozent Glycerinmonolaurinsäureester,

(B) 0,2 bis 10 Gewichtsprozent eines geradekettigen Fettalkohols mit 10 bis 18 Kohlenstoffatomen, oder eines Gemisches solcher Fettalkohole,

(C) 0,005 bis 5 Gewichtsprozent einer quaternären Verbindung der allgemeinen Formel

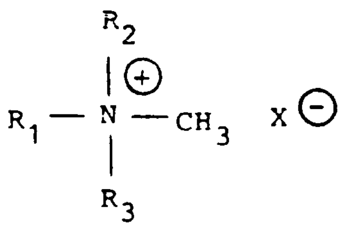

oder

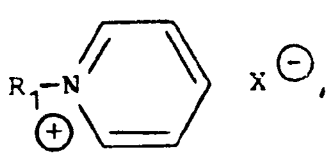

wobei $R_1$ einen Alkylrest mit 12 bis 22 Kohlenstoffatomen, Kokosamidoethyl, Kokosamidopropyl, R′-Y- (mit R′ = $C_{12}$ bis $C_{20}$-Alkyl und

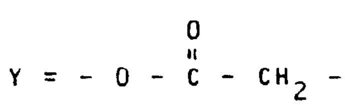

oder

$$- \overset{\displaystyle O}{\overset{\|}{C}} - O - CH_2 - CH_2 -)$$

darstellt,
$R_2$ gleich $CH_3$, $CH_2CH_2OH$ oder $CH_2CH_3$ ist,
$R_3$ einen der Reste $CH_3$, $CH_2CH_2OH$, Benzyl oder Acetamidyl bedeutet und
X = Cl, Br, $CH_3SO_4$, 1/2 $SO_4$, Dihydrogenphosphat, Lactat oder Acetat ist,
(D) 70 bis 99,595 Gewichtsprozent Wasser und
(E) 0 bis 24 Gewichtsprozent üblichen kosmetischen Zusatzstoffen, wobei
(1) der Gewichtsanteil der kosmetischen Zusatzstoffe nicht größer ist als der Gewichtsanteil aus der Summe der Komponenten (A), (B) und (C),
(2) das Gewichtsverhältnis der Komponente (A) zur Komponente (B) 0,3 bis 5 ist und
(3) das Gewichtsverhältnis der Komponente (C) zur Summe der Komponenten (A) und (B) 0,01 bis 0,4 beträgt.

Das erfindungsgemäße Haarkonditionierungsmittel weist eine sehr gute kämmbarkeitsverbessernde Wirkung, eine gleichbleibende Viskosität, ein stabiles perlglänzendes Aussehen sowie eine gute Lagerfähigkeit auf.

Als Glycerinmonolaurinsäureester der Komponente (A) können nur solche Handelsprodukte verwendet werden, die einen Reinheitsgrad von mindestens 90 % besitzen.

Besonders vorteilhaft ist es hierbei, wenn der Glycerinmonolaurinsäureester der Komponente (A) in einer Menge von 0,5 bis 4 Gewichtsprozent in dem erfindungsgemäßen Mittel enthalten ist.

Die besonders bevorzugte Einsatzmenge für den Fettalkohol der Komponente (B) beträgt 0,8 bis 5 Gewichtsprozent. Als Fettalkohole kommen alle geradkettigen Fettalkohole mit 10 bis 18 Kohlenstoffatomen, wie zum Beispiel Laurylalkohol, Tetradecanol, Cetylalkohol und Stearylalkohol, in Betracht. Die Komponente (B) kann ferner aus einem Gemisch dieser Fettalkohole, beispielsweise aus einem 1 : 1 Gemisch von Cetylalkohol und Stearylalkohol (Handelsprodukt LANETTE® O der Firma Henkel, Düsseldorf), bestehen.

Als quaternäre Verbindungen der Komponente (C) sind vor allem quaternäre Ammoniumverbindungen, wie zum Beispiel Lauryltrimethylammoniumchlorid, Tetradecyltrimethylammoniumchlorid, Cetyltrimethylammoniumchlorid, Stearyltrimethylammoniumchlorid, Cetyldimethylhydroxyethylammoniumdihydrogenphosphat, Cetyldimethylbenzylammoniumchlorid, ferner Pyridiniumsalze, wie beispielsweise Cetylpyridiniumchlorid sowie andere quaternäre Verbindungen, wie zum Beispiel Laurylsäurecholinesterchlorid, Betaincetylesterchlorid und Kokosfettsäureamidopropyldimethylacetamidylammoniumchlorid geeignet. Vorzugsweise sind die quaternären Verbindungen der Komponente (C) in dem erfindungsgemäßen Mittel in einer Menge von 0,1 bis 1,5 Gewichtsprozent enthalten.

Die Hauptmenge des erfindungsgemäßen Mittels besteht aus Wasser, wobei der Wassergehalt vorzugsweise 90 bis 97 Gewichtsprozent beträgt. Obwohl Leitungswasser verwendet werden kann, wenn es eine relativ geringe Menge an Ionen enthält, ist es vorteilhaft, entionisiertes ("vollentsalztes") Wasser zu verwenden.

Das hier beschriebene Haarkonditionierungsmittel kann darüberhinaus alle diejenigen kosmetischen Zusatzstoffe enthalten, die üblicherweise in Haarkonditionierungsmitteln eingesetzt werden, insbesondere Farbstoffe, Parfümöle, Vitamine, Konservierungsstoffe, Antioxidantien sowie Antischuppenwirkstoffe. Die vorstehend genannten Zusatzstoffe können jeweils in einer Menge von etwa 0,1 bis 1 Gewichtsprozent enthalten sein. Weitere übliche Zusatzstoffe sind zum Beispiel kosmetische öle, wie beispielsweise Avocadoöl oder Pflegestoffe, wie zum Beispiel Lanolin, Cholesterin und Panthothensäure, wobei diese Zusatzstoffe in einer Menge von etwa 0,1 bis 10 Gewichtsprozent enthalten sein können.

Das Mittel kann als Zusatzstoffe ferner kationische Harze sowie Verdicker, beispielsweise Stärke, Cellulosederivate oder hochdisperse Kieselsäure (zum Beispiel Aerosil® 300 der Firma Degussa, Hanau, Bundesrepublik Deutschland) in einer Menge von etwa 0,1 bis 2 Gewichtsprozent enthalten.

Das erfindungsgemäße Haarkonditionierungsmittel kann auch in Form eines Aerosolpräparates vorliegen und enthält dann als weiteren üblichen Zusatzstoff etwa 1 bis 10 Gewichtsprozent eines Treibmittels. Als Treibmittel können Chlorfluoralkane, wie zum Beispiel $CCl_3F$, $CCl_2F_2$, $C_2Cl_3F_3$, $CCl_2F$-$CCl_2F$, $CHCl_2F$, $CHClF_2$ und $CClF_2$-$CClF_2$, leichtflüchtige Kohlenwasserstoffe, wie beispielsweise n-Butan, i-Butan und n-Propan, oder auch Dimethylether, $CO_2$, $N_2O$, $N_2$, $CH_2Cl_2$ und $CCl_3$-$CH_3$ sowie Mischungen der vorstehend genannten Stoffe Verwendung finden.

Die Herstellung des erfindungsgemäßen Mittels erfolgt üblicherweise nach den für kosmetische Emulsionen bekannten Herstellungsverfahren, indem zum Beispiel die hydrophoben Bestandteile (Komponente (A) und (B) sowie gegebenenfalls enthaltene hydrophobe Zusatzstoffe) zunächst bei 70 bis 90 Grad Celsius

geschmolzen werden. Sodann werden die hydrophilen Bestandteile (Komponente (C) sowie hydrophile Zusatzstoffe) in Wasser gelöst, die so erhaltene wäßrige Lösung auf 70 bis 90 Grad Celsius erwärmt und in die Schmelze der hydrophoben Bestandteile unter Rühren einemulgiert. Schließlich wird die entstandene Emulsion abgekühlt.

Die nachstehenden Beispiele sollen den Gegenstand der Erfindung näher erläutern, ohne den Gegenstand auf diese Beispiele zu beschränken.

**Beispiele**

**Beispiele 1 bis 5:**

Einfluß des Glycerinfettsäureesters auf die Perlglanzbildung

| Beispiel (Mengenangaben in Gramm) | 1 | 2 | 3 | 4 | 5 |
|---|---|---|---|---|---|
| (A) Glycerinmonolaurinsäureester | - | - | 1,24 | 2,48 | 2,14 |
| Glycerinmonostearinsäureester | - | 2,14 | 1,24 | - | - |
| Glycerinmonodistearinsäureester | 5,00 | - | - | - | - |
| (B) Stearylalkohol | 3,36 | 1,43 | 2,07 | 2,07 | 1,43 |
| (C) Stearyltrimethylammoniumchlorid | 2,62 | 1,43 | 0,45 | 0,45 | 1,43 |
| Farbstoff | 1,00 | 1,00 | 1,00 | 1,00 | 1,00 |
| Parfümöl | 0,50 | 0,50 | 0,50 | 0,50 | 0,50 |
| Wasser (vollentsalzt) | 87,52 | 93,50 | 93,50 | 93,50 | 93,50 |
| | 100,00 | 100,00 | 100,00 | 100,00 | 100,00 |
| (A) : (B) = | ./. | ./. | 0,60 | 1,20 | 1,50 |
| (C) : (A) + (B) = | ./. | ./. | 0,14 | 0,10 | 0,40 |
| Perlglanz | - | - | + | + | + |

Die hydrophoben Komponenten werden bei 80 Grad Celsius geschmolzen und mit der auf 80 Grad Celsius erwärmten wäßrigen Lösung der wasserlöslichen Komponenten unter Rühren emulgiert. Nach dem Abkühlen erhält man bei den Mitteln gemäß den Beispielen 1 und 2 lediglich trübe Emulsionen, während die erfindungsgemäßen Mittel (Beispiel 3 bis 5) einen Perlglanzeffekt aufweisen. Die Beispiele 1 bis 5 beweisen somit eindeutig, daß nur bei Verwendung von Glycerinmonolaurinsäureester, nicht jedoch bei Verwendung anderer Glycerinfettsäureester, ein Haarkonditionierungsmittel mit Perlglanz erhalten wird.

**Beispiel 6 bis 10**

Einfluß des Gewichtsverhältnisses von (A) zu (B) und (C) zu (A) + (B) auf die Lagerstabilität des Haarkondi-

tionierungsmittels

| Beispiel (Mengenangaben in Gramm) | 6 | 7 | 8 | 9 | 10 |
|---|---|---|---|---|---|
| (A) Glycerinmonolaurinsäureester | 1,33 | 7,11 | 3,33 | 6,60 | 1,98 |
| (B) Stearylalkohol | 6,67 | 0,89 | 1,67 | 3,30 | 2,37 |
| (C) Stearyltrimethylammoniumchlorid | 2,00 | 2,00 | 5,00 | 0,08 | 0,65 |
| Parfümöl | 0,50 | 0,50 | 0,50 | 0,50 | 0,50 |
| Wasser (vollentsalzt) | 89,50 | 89,50 | 89,50 | 89,52 | 94,50 |
| | 100,00 | 100,00 | 100,00 | 100,00 | 100,00 |

| | | | | | |
|---|---|---|---|---|---|
| (A) : (B) = | 0,20 | 7,99 | 1,99 | 2,00 | 0,84 |
| (C) : (A) + (B) = | 0,25 | 0,25 | 1,00 | 0,008 | 0,15 |
| Perlglanz | o | o | - | + | + |
| Lagerstabilität (3 Monate bei 20° C) | instabil, Separation | instabi, Separation | ./. | instabil, Separation | stabil, keine Separation |

+ = Perlglanz
o = schwacher Perlglanz
- = keine Perglanz

Wie die vorstehenden Beispiele zeigen, wird ein lagerstabiles, das heißt während der Lagerung nicht separierendes, perlglänzendes Mittel nur dann erhalten, wenn das Gewichtsverhältnis von (A) zu (B) und (C) zu (A) + (B) wie in Beispiel 10 innerhalb des erfindungsgemäßen Bereichs liegt.

**Beispiel 11 bis 19:**

Lagerstabiles Haarkonditionierungsmittel mit Perlglanz

Die Beispiele 11 bis 19 zeigen die Verwendbarkeit verschiedener quaternärer Verbindungen und verschiedener Fettalkohole, wobei ebenfalls die Einsatzmengen der Komponenten (A), (B) und (C) variieren.

Die nachstehend aufgeführten Haarkonditionierungsmittel werden in der bei den Beispielen 1 bis 5 beschriebenen Weise hergestellt. Diese Haarkonditionierungsmittel zeigen auch nach längerer Lagerung einen stabilen Perlglanz, wobei das Mittel gemäß Beispiel 12 auch nach mehrmonatiger Lagerung bei 40° C einen stabilen Perlglanz aufweist.

| Beispiel (Mengenangaben in Gramm) | 11 | 12 |
|---|---|---|
| (A) Glycerinmonolaurinsäureester | 2,37 | 2,37 |
| (B) Stearylalkohol | - | 1,98 |
| (B) Cetylstearylalkohol | 1,98 | - |
| (C) Stearyltrimethylammoniumchlorid | 0,65 | 0,65 |
| Parfümöl | 0,50 | 0,50 |
| Wasser (vollentsalzt) | 94,50 | 94,50 |
| | 100,00 | 100,00 |
| (A) : (B) = | 1,20 | 1,20 |
| (C) : [(A) + (B)] = | 0,15 | 0,15 |

| Beispiel (Mengenangaben in Gramm) | 13 | 14 | 15 |
|---|---|---|---|
| (A) Glycerinmonolaurinsäureester | 2,37 | 2,37 | 2,37 |
| (B) Stearylalkohol | 1,98 | 1,98 | 1,98 |
| (C) Cetyldimethylbenzylammoniumchlorid | 0,65 | - | - |
| (C) Lauryltrimethylammoniumchlorid | - | 0,65 | - |
| (C) $C_{20}$ - $C_{22}$-Alkyltrimethylammoniumchlorid | - | - | 0,65 |
| Farbstoff | 1,00 | 1,00 | 1,00 |
| Wasser (vollentsalzt) | 93,50 | 93,50 | 93,50 |
| | 100,00 | 100,00 | 100,00 |
| (A) : (B) | 1,20 | 1,20 | 1,20 |
| (C) : [(A) + (B)] | 0,15 | 0,15 | 0,15 |

| Beispiel (Mengenangaben in Gramm) | 16 | 17 | 18 |
|---|---|---|---|
| (A) Glycerinmonolaurinsäureester | 1,98 | 2,48 | 6,60 |
| (B) Stearylalkohol | 2,37 | 2,07 | 3,30 |
| (C) Stearyltrimethylammoniumchlorid | 0,65 | 0,45 | 0,10 |
| Farbstoff | 1,00 | 1,00 | 1,00 |
| Parfümöl | 0,50 | 0,50 | 0,50 |
| Wasser (vollentsalzt) | 93,50 | 93,50 | 88,50 |
| | 100,00 | 100,00 | 100,00 |
| (A) : (B) = | 0,84 | 1,20 | 2,00 |
| (C) : [(A) + (B)] = | 0,15 | 0,10 | 0,01 |

Jeweils 20 g der vorstehenden Haarkonditionierungsmittel werden im gewaschenen, handtuchtrockenen Haar verteilt und nach einer Einwirkungszeit von 3 bis 5 Minuten mit lauwarmem Wasser ausgespült. Das so behandelte Haar besitzt eine gute Kämmbarkeit und einen ausgezeichneten Griff.

**Beispiel 19:**

Haarkonditionierungsmittel mit Perlglanz (Haarkur mit Body-Effekt)

| | |
|---|---|
| (A) Glycerinmonolaurinsäureester | 1,98 g |
| (B) Stearylalkohol | 2,37 g |
| (C) Stearyltrimethylammoniumchlorid | 0,65 g |
| hochdisperse Kieselsäure (Aerosil® 300 der Firma Degussa) | 0,50 g |
| Parfümöl | 0,50 g |
| Wasser (vollentsalzt) | 94,00 g |
| | 100,00 g |
| (A) : (B) = | 0,84 g |
| (C) : [(A) + (B)] = | 0,15 g |

Die vorstehende Haarkur wird in der in den Beispielen 11 bis 18 beschriebenen Weise hergestellt und angewendet. Geschädigtes Haar zeigt nach der Anwendung dieser Haarkur eine gute Kämmbarkeit sowie eine größere Fülle (Body-Effekt).

**Beispiel 20:**

Herstellung von Haarkonditionierungsmitteln mit Perlglanz

Komponente I

| | | |
|---|---|---|
| (A) | Glycerinmonolaurinsäureester | 10,00 g |
| (C) | Stearyltrimethylammoniumchlorid | 2,62 g |
| | Farbstoff | 1,00 g |
| | Parfümöl | 0,50 g |
| | Wasser | 85,88 g |
| | | 100,00 g |

| Komponente II | | (a) | (b) |
|---|---|---|---|
| (B) | Cetylstearylalkohol | 4,0 g | 6,0 g |
| (C) | Trimethylhexadecyl-ammoniumchlorid | 1,0 g | 0,5 g |
| | Parfümöl | 0,5 g | 0,5 g |
| | Wasser | 94,5 g | 93,0 g |
| | | 100,0 g | 100,0 g |

Die Komponente I wird in der Kälte mit der Komponente IIa beziehungsweise IIb in einem Verhältnis von 1 zu 5 vermischt. Es wird ein stabiles, perlglänzendes Haarkonditionierungsmittel mit sehr guten haarpflegenden Eigenschaften erhalten.

In den Beispielen 1 bis 20 wurde als Glycerinmonolaurinsäureester ein handelsübliches Produkt, dessen Gehalt an Glycerinmonolaurinsäureester mindestens 90 Gewichtsprozent beträgt, eingesetzt.

Die Prozentangaben in dieser Anmeldung stellen, soweit nicht anders vermerkt, Gewichtsprozent dar.

**Patentansprüche**

1. Lagerstabiles, nicht separierendes Haarkonditionierungsmittel mit Perlglanz auf der Basis eines Glycerinfettsäureesters, einer quaternären Verbindung sowie eines Fettalkohols, bestehend aus

(A) 0,2 bis 10 Gewichtsprozent Glycerinmonolaurinsäureester,

(B) 0,2 bis 10 Gewichtsprozent eines geradkettigen Fettalkohols mit 10 bis 18 Kohlenstoffatomen, oder eines Gemisches solcher Fettalkohole,

(C) 0,005 bis 5 Gewichtsprozent einer quaternären Verbindung der allgemeinen Formel

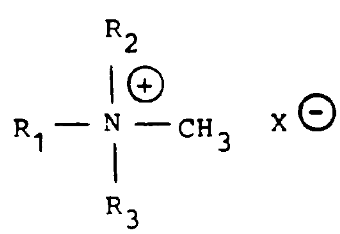

oder

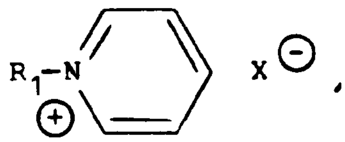

wobei $R_1$ einen Alkylrest mit 12 bis 22 Kohlenstoffatomen, Kosamidoethyl, Kokosamidopropyl, R′-Y- (mit R′ = $C_{12}$ bis $C_{20}$-Alkyl und

$$Y = -O-\overset{O}{\overset{\|}{C}}-CH_2-$$

oder

$$-\overset{O}{\overset{\|}{C}}-O-CH_2-CH_2-)$$

darstellt,

$R_2$ gleich $CH_3$, $CH_2CH_2OH$ oder $CH_2CH_3$ ist,

$R_3$ einen der Reste $CH_3$, $CH_2CH_2OH$, Benzyl oder Acetamidyl bedeutet und

x= Cl, Br, $CH_3SO_4$, 1/2 $SO_4$, Dihydrogenphosphat, Lactat oder Acetat ist,

(D) 70 bis 99,595 Gewichtsprozent Wasser und

(E) 0 bis 24 Gewichtsprozent üblichen kosmetischen Zusatzstoffen

wobei

(1) der Gewichtsanteil der kosmetischen Zusatzstoffe nicht größer ist, als der Gewichtsanteil aus der Summe der Komponenten (A), (B) und (C),

(2) das Gewichtsverhältnis der Komponente (A) zur Komponente (B) 0,3 bis 5 ist und

(3) das Gewichtsverhältnis der Komponente (C) zur Summe der Komponenten (A) und (B) 0,01 bis 0,4 beträgt.

2. Mittel nach Anspruch 1, dadurch gekennzeichnet, daß die Komponente (A) in einer Menge von 0,5 bis 4 Gewichtsprozent enthalten ist.

3. Mittel nach Anspruch 1 oder 2, dadurch gekennzeichnet, daß der Fettalkohol der Komponente (B) ausgewählt ist aus Laurylalkohol, Tetradecanol, Cetylalkohol, Stearylalkohol und einem 1 : 1 Gemisch von Cetylalkohol und Stearylalkohol.

4. Mittel nach einem der Ansprüche 1 bis 3, dadurch gekennzeichnet, daß die Komponente (B) in einer Menge von 0,8 bis 5 Gewichtsprozent enthalten ist.

5. Mittel nach einem der Ansprüche 1 bis 4, dadurch gekennzeichnet, daß die quaternäre Verbindung der Komponente (C) ausgewählt ist aus Lauryltrimethylammoniumchlorid, Tetradecyltrimethylammoniumchlorid, Cetyltrimethylammoniumchlorid, Stearyltrimethylammoniumchlorid, Cetyldimethylbenzylammoniumchlorid, Cetylpyridiniumchlorid, Cetyldimethylhydroxyethylammoniumdihydrogenphosphat, Laurylsäurecholinesterchlorid, Betaincetylesterchlorid und Kokosfettsäureamidopropyldimethylacetamidylammoniumchlorid.

6. Mittel nach einem der Ansprüche 1 bis 5, dadurch gekennzeichnet, daß die Komponente (C) in einer Menge von 0,1 bis 1,5 Gewichtsprozent enthalten ist.

7. Mittel nach einem der Ansprüche 1 bis 6, dadurch gekennzeichnet, daß es 90 bis 97 Gewichtsprozent Wasser enthält.

8. Mittel nach einem der Ansprüche 1 bis 7, dadurch gekennzeichnet, daß der kosmetische Zusatzstoff der Komponente (E) ausgewählt ist aus kosmetischen Farbstoffen, Parfümölen, Konservierungsmitteln, Vitaminen, Antioxidantien, Verdickern, kosmetischen ölen, Pflegestoffen, kationischen Harzen und Antischuppenwirkstoffen.

9. Mittel nach einem der Ansprüche 1 bis 8, dadurch gekennzeichnet, daß es 1 bis 10 Gewichtsprozent eines Treibmittels enthält und in Form eines Aerosolpraparates vorliegt.

## Claims

1. Non-separating hair conditioning agent which is stable during storage, has a pearly lustre and is based on a glycerol fatty acid ester, and a quaternary compound as well as on a fatty alcohol, consisting of:

(A) 0.2 to 10 wt.% glycerolmonolauric acid ester;

(B) 0.2 to 10 wt.% of a straight-chain fatty alcohol with 10 to 18 carbon atoms, or a mixture of such fatty alcohols;

(C) 0.005 to 5 wt.% of a quaternary compound having the general formula:

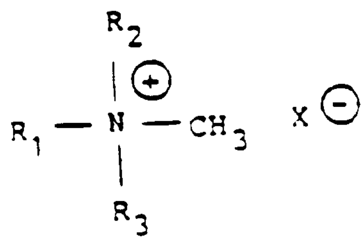

or

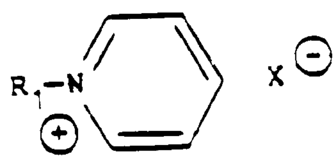 ,

wherein $R_1$ is an alkyl residue with 12 to 22 carbon atoms, coconut amidoethyl, coconut amidopropyl, R'-Y-(where R'=$C_{12}$ to $C_{20}$ alkyl and

Y = 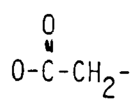

OR

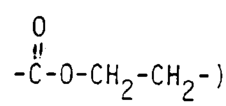

$R_2$ is $CH_3$, $CH_2CH_2OH$ or $CH_2CH_3$,

$R_3$ is a $CH_3$, $CH_2CH_2OH$, benzyl or acetamidyl residue and

X = Cl, Br, $CH_3SO_4$, 1/2 $SO_4$ dihydrogenphosphate, lactate or acetate,

(D) 70 to 99.595 wt.% water and

(E) 0 to 24 wt.% of conventional cosmetic additives,

wherein

(1) the weight proportion of the cosmetic additives is not greater than the weight proportion of the total of components (A), (B) and (C);

(2) the weight ratio of component (A) to component (B) is 0.3 to 5; and

(3) the weight ratio of component (C) to the sum of components (A) and (B) is 0.01 to 0.4.

2. Agent according to Claim 1, characterised in that component (A) is present in an amount of between 0.5 and 4 wt.%.

3. Agent according to Claim 1 or 2, characterised in that the fatty alcohol of component (B) is selected from lauryl alcohol, tetradecanol, cetyl alcohol, stearyl alcohol, and a 1:1 mixture of cetyl alcohol and stearyl alcohol.

4. Agent according to any one of Claims 1 to 4, characterised in that component (B) is present in an amount of between 0.8 and 5 wt.%.

5. Agent according to any one of Claims 1 to 4, characterised in that the quaternary compound of component (C) is selected from lauryltrimethylammoniumchloride, tetradecyltrimethylammoniumchloride, cetyltrimethylammoniumchloride, stearyltrimethylammoniumchloride, cetyldimethylbenzylammoniumchloride, cetylpyridiniumchloride, cetyldimethylhydroxyethylammoniumdihydrogenphosphate, lauric acid cholinesterchloride, betaincetylesterchloride and coconut fatty acid amidopropyldimethylacetamidylammoniumchloride.

6. Agent according to any one of claims 1 to 5, characterised in that component (C) is present in an amount of between 0.1 and 1.5 wt.%.

7. Agent according to any one of Claims 1 to 6, characterised in that it contains 90 to 97 wt. % water.

8. Agent according to any one of Claims 1 to 7, characterised in that the cosmetic additive of component (E) is selected from cosmetic dyes, perfume oils, preservatives, vitamins, antioxidants, thickeners, cosmetic oils, treatment substances, cationic resins and anti-dandruff agents.

9. Agent according to any one of Claims 1 to 8, characterised in that it contains 1 to 10 wt.% of a propellant and is in the form of an aerosol preparation.

## Revendications

1. Agent de conditionnement capillaire ne se décantant pas, stable au stockage, donnant un éclat nacré, à base d'un ester d'acide gras glycérique, d'un composé quaternaire ainsi que d'un alcool gras, se composant de:

(A) 0,2 à 10% en poids d'un ester d'acide glycérique monolaurique,

(B) 0,2 à 10% en poids d'un alcool gras à chaîne droite comportant 10 à 18 atomes de carbone, ou un mélanges de tels alcools gras.

(C) 0,005 à 5% en poids d'un composé quaternaire de formule générale:

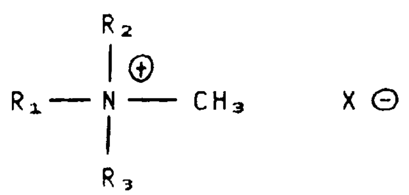

ou

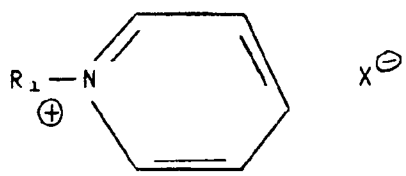

dans laquelle R1 est un reste alkyle, comprenant 12 à 22 atomes de carbone, du kokosamidoéthyl, du kokosamidopropyle, R'-Y (R' étant un groupe alkyle en $C_{12}$ à $C_{20}$) et

$$Y = -O-\overset{\overset{O}{||}}{C}-CH_2$$

ou

$$-\overset{\overset{O}{||}}{C}-O-CH_2-CH_2-)\ ,$$

$R_2$ est égal à $CH_3$,$CH_2CH_2OH$, ou $CH_2CH_3$

$R_3$ représente un des restes $CH_3$, $CH_2CH_2OH$, benzyle, ou, acétamidyle,

X = Cl, Br, $CH_3SO_4$, 1/2 de $SO_4$, dihydrogénophosphate, lactate ou acétate

(D) va de 70 à 99,595% en poids d'eau,

(E) va de 0 à 24% d'additifs cosmétiques usuels,

où

(1) la proportion en poids d'additifs cosmétiques n'est pas supérieure à la proportion en poids de la somme des composants (A), (B) et (C).

(2) Le rapport pondéral des composants (A) par rapport aux composants (B) va de 0,3 à 5,

(3) Le rapport pondéral des composants (C) par rapport à la somme des composants (A) et (B), va de 0,01 à 0,4.

2. Agent selon la revendication 1, caractérisé en ce que le composant (A) est présent à raison de 0,5 à 4 % en poids.

3. Agent selon la revendication 1 ou 2, caractérisé en ce que l'alcool gras du composant (B) est choisi parmi l'alcool laurique, le tétradécanol, l'alcool cétylique, l'alcool stéarylique et un mélange 1 : 1 d'alcool cétylique et d'alcool stéarylique.

4. Agent selon l'une des revendications 1 à 3, caractérisé en ce que le composant (B) est présent à raison de 0,8 à 5% en poids.

5. Agent selon l'une des revendications 1 à 4, caractérisé en ce que le composé quaternaire du composant (C) est choisi parmi le chlorure de lauryltriméthylammonium, le chlorure de tétradécyltriméthylammonium, le chlorure de cétyltriméthylammonium, le chlorure de cétyl-diméthyl- benzylammonium, le chlorure de cétyl-pyridinium, le dihydrogénophosphate de cétyl-diméthyl-hydroxyéthylammonium, le chlorure d'ester cholique de l'acide laurique, le chlorure de stéaryltriméthylammonium, le chlorure de l'ester cétylique de la bétaïne et le chlorure d'amidopropyldiméthylacétamidylammonium de l'acide d'huile de coco.

6. Agent selon l'une des revendications 1 a 5, caractérisé en ce que le composant (C) est présent à raison de 0,1 à 1,5% en poids.

7. Agent selon l'une des revendications 1 à 6, caractérisé en ce qu'il contient de 90 à 97% d'eau.

8. Agent selon l'une des revendications 1 à 7, caractérisé en ce que l'additif cosmétique du composant (E) est choisi parmi les colorants cosmétiques, les huiles essentielles, les agents de conservation, les vitamines, les antioxydants, les huiles cosmétiques, les épaississants, les produits de soins capillaires, les matières résineuses cationiques et anti-écaillage.

9. Agent selon l'une des revendications 1 à 8, caractérisé en ce qu'il contient 1 à 10% en poids d'un agent propulseur, et qu'il se présente sous forme d'une préparation d'aérosol.